# EUROPEAN PATENT SPECIFICATION

(11) **EP 1 256 585 B1**
(45) Date of publication and mention of the grant of the patent: **29.09.2004**
(21) Application number: 02016429.9
(22) Date of filing: 23.07.1998
(51) Int. Cl.: C07F 9/6561

(54) **Process for preparing 9-(2-(diethylphosphonomethoxy)ethyl)-adenine**
Verfahren zur Herstellung von 9-(2-(diethylphosphonomethoxy)ethyl)-adenin
Procédé de préparation de la 9-(2-(diéthylphosphonomethoxy)éthyl)-adénine

(30) Priority: 25.07.1997 US 900745; 25.07.1997 US 53771 P
(43) Date of publication of application: 13.11.2002
(62) Divisional of application: 98937988.8
(73) Proprietor: GILEAD SCIENCES, INC., Foster City CA 94404 (US)
(72) Inventor: Arimilli, Murty N, Fremont, California 94555 (US); Lee, Thomas, T.K, Redwood City, California 94065 (US); Manes, Lawrence V, Moss Beach, California 94038 (US); Munger, John D.,Jr, Alviso, California 95002 (US); Prisbe, Ernest J, Los Altos, California 94024 (US); Schultze, Lisa, M, Woodinville, Washington 98072 (US); Kelly, Daphne E, San Mateo, California 94401 (US)
(74) Representative: Kinzebach, Werner, Dr.

(56) References cited:
- EP-A- 0 253 412
- US-A- 5 514 798
- CHEN, WEI ET AL: "An improved synthesis of 9-[2-(diethoxyphosphonomethoxy) ethyl]adenine and its analogs with other purine bases utilizing the Mitsunobu reaction" NUCLEOSIDES & NUCLEOTIDES (1996), 15(11 & 12), 1771-1778 , 1996, XP002210045
- HOLY, ANTONIN ET AL: "Acyclic nucleotide analogs. Part III. Synthesis of 9-(2-phosphonylmethoxyethyl)adenine and related compounds" COLLECT. CZECH. CHEM. COMMUN. (1987), 52(11), 2801-9 , 1987, XP002210046
- SCHULTZE L M ET AL: "Practical Synthesis of the anti-HIV Drug, PMPA" TETRAHEDRON LETTERS, ELSEVIER SCIENCE PUBLISHERS, AMSTERDAM, NL, vol. 39, no. 14, 2 April 1998 (1998-04-02), pages 1853-1856, XP004109567 ISSN: 0040-4039

## Description

The invention relates to a method for preparing 9-[2-(diethylphosphonomethoxy)ethyl] adenine. The bis-pivaloyloxymethyl ester of the compound 9-[2-(phosphonomethoxy)ethyl] adenine ("PMEA") is the nucleotide analog 9-[2[[bis [ (pivaloyloxy)-methoxy]phosphinyljmethoxyethyl] adenine ("adefovir dipivoxil" or "AD").

AD has antiviral activity in animals and in humans. AD and PMEA have been described, e.g., U.S. Patent Numbers 4,724,233 and 4,808,716, EP 481 214, Benzaria et al., Nucleosides and Nucleotides (1995) 14(3-5):563-565, Holy et al., Collect. Czech. Chem. Commun. (1989) 54:2190-2201, Holy et al., Collect. Czech. Chem. Commun. (1987) 52:2801-2809. Rosenberg et al., Collect. Czech. Chem. Commun. (1988) 53:2753-2777, Starrett et al., Antiviral Res. (1992) 19:267-273; Starrett et al., J. Med. Chem. (1994) 37:1857-1864.

US-A 5,514,798 discloses a method for preparing 9-[2-(diethylphosphonomethoxy)ethyl] adenine by reacting 9-(2-hydroxyethyl)-adenine with sodium hydride in anhydrous DMF and adding thereto a solution of diethyl toluenesulfonyloxymethylphosphonate.

The invention provides a method for preparing 9-[2-(diethylphosphonomethoxy)ethyl] adenine comprising the step of contacting sodium alkoxide and 9-(2-hydroxyethyl)-adenine.

Preferably, 1.2 - 2.2 molar equivalents of sodium alkoxide per molar equivalent of 9-(2-hydroxyethyl)-adenine are used.

Preferably, the sodium alkoxide and 9-(2-hydroxyethyl)-adenine are contacted in dimethylformide as solvent.

Preferably, the sodium alkoxide and 9-(2-hydroxyethyl)-adenine are contacted at a temperature of 20 to 30 °C.

Preferably, the sodium alkoxide is selected from sodium t-butoxide and sodium i-propoxide.

In a typical embodiment, the method further comprises the step of reacting the obtained product with diethyl p-toluenesulfonyloxymethylphosphonate.

### Methods for AD Synthesis

Diagram A below shows a representative process flow diagram for making AD and Form 1 AD crystals.

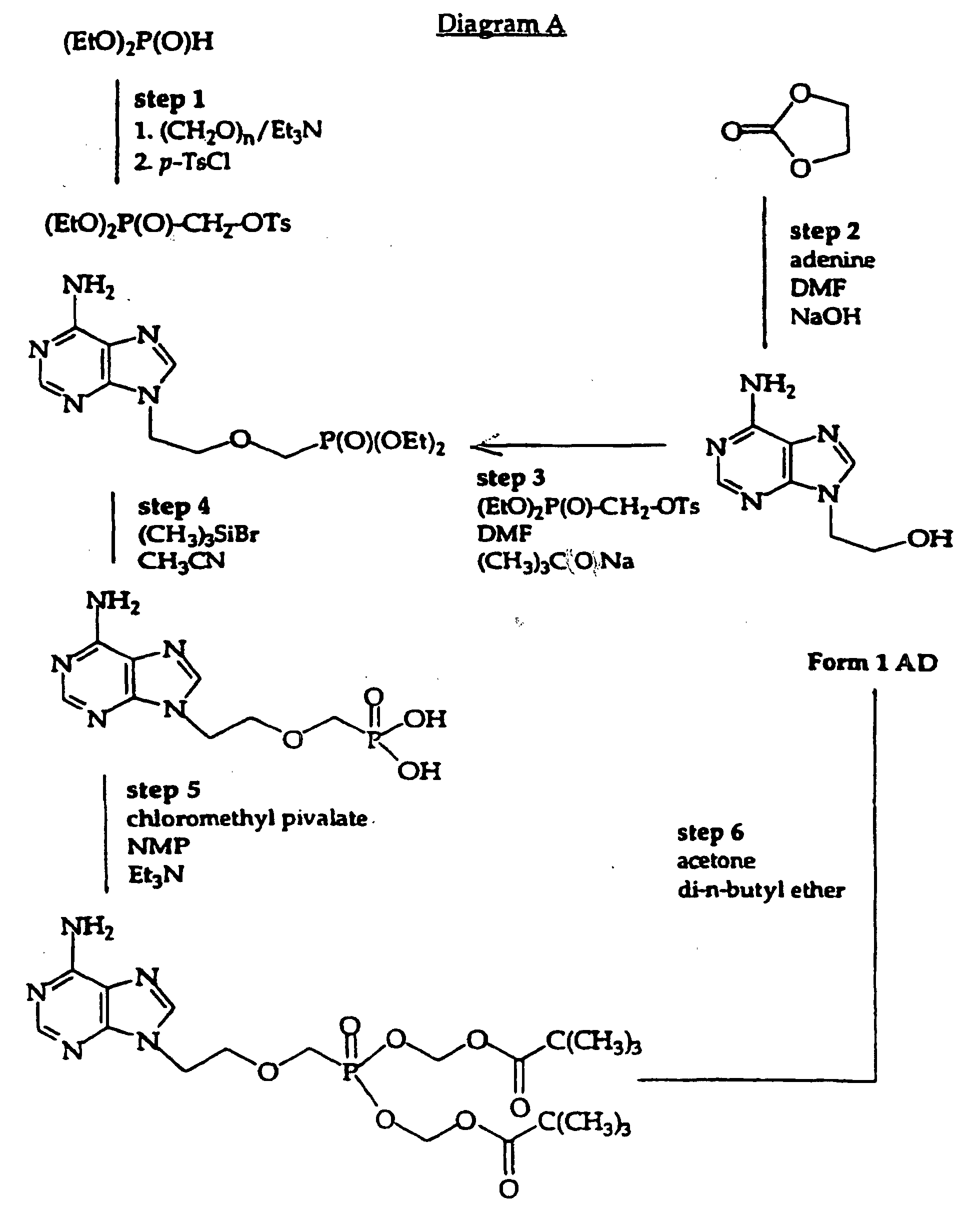

One can increase or decrease the scale of the process steps shown in Diagram A and described below if desired.

### Methods for Diethyl p-toluenesulfonyloxymethylphosphonate Synthesis

In an embodiment, synthesis of diethyl *p*-toluenesulfonyloxymethyl-phosphonate, shown in Diagram A, Step 1, is described as follows. In a reactor having an inert atmosphere, e.g., nitrogen, a mixture of diethylphosphite (0.8 kg), paraformaldehyde (0.22 kg), and triethylamine (0.06 kg) in toluene (2.69 kg) is heated to 87°C (84 to 110°C) for 2 hours with agitation, then heated to reflux and maintained for at reflux for 1 hour, until the reaction is complete. Reaction completion is monitored by TLC (trace or no diethyl phosphite detectable) and confirmed by ¹H NMR showing no more than 1% of the diethyl phosphite peak at δ 8.4-8.6 ppm. The solution is cooled to about 1°C (-2 to 4°C) and *p*-toluenesulfonyl chloride (1.0 kg) is added and then triethylamine (0.82 kg) at no more than 10°C is slowly added (over about 3-6 hours in an exothermic reaction). The resulting mixture is warmed to 22°C (19-25°C) and stirred for at least 5 hours (typically about 16-24 hours), until the reaction is complete. Reaction completion is monitored by TLC (trace or no *p*-toluenesulfonyl chloride detectable) and confirmed by ¹H NMR (*p*-toluenesulfonyl chloride doublet at *δ* 7.9 ppm no longer detected). The solids are removed by filtration and rinsed with toluene (0.34 kg). The combined washings and filtrate are washed either twice with water (1.15 kg each), or optionally with a sequence of water (1.15 kg), 5% aqueous sodium carbonate (3.38 kg), and twice with water (1.15 kg each). In the event emulsion occurs, brine may be added to the first organic/water mixture. The organic phase, which is at no more than 50°C, is distilled *in vacuo* (to LOD no more than 10% and water content, by KF (Karl Fischer) titration, no more than 0.5%), affording the title compound as an oil of about 85-95% purity, exclusive of toluene. The oil may become viscous on cooling.

### Methods for 9-(2-Hydroxyethyl)adenine Synthesis

In an embodiment, synthesis of 9-(2-hydroxyethyl)adenine, shown in Diagram A, Step 2, is described as follows. In a reactor having an inert atmosphere, e.g., nitrogen, sodium hydroxide (6 g) is added to a slurry of adenine (1.0 kg) and molten ethylene carbonate (0.72 kg, m.p. 37-39°C), in DMF (2.5 kg) and the mixture is heated to 125°C (95°C to reflux) with agitation until the reaction is complete (about 3-9 hours if the mixture temperature is at 110°C to reflux or about 15-48 hours if at 95 to 110°C). Reaction completion is monitored by HPLC (no more than 0.5% adenine remaining). The mixture is cooled to below 50°C and diluted with toluene (3.2 kg). The resulting slurry is cooled to 3°C (0-6°C) and agitated for at least 2 hours. The slurry is filtered and the filter cake is washed twice with cold (0-5°C) toluene (0.6 kg each). The filter cake is dried *in vacuo* at 35 to 70°C (no more than 2% toluene, by ¹H NMR or LOD) and optionally milled, affording the title compound as a white to off-white powdery solid.

### Methods for 9-[2-(Diethylphosphonomethoxy)ethylladenine Synthesis

This compound is prepared using a composition comprising sodium alkoxide (C₁₋₆ alkyl) and 9-(2-hydroxyethyl)adenine. One contacts sodium alkoxide, typically sodium t-butoxide or sodium i-propoxide, with 9-(2-hydroxyethyl)adenine in a solvent such as DMF, at a temperature of about 20-30° over about 1-4 hours. The synthesis typically gives good results with 1 molar equivalent of 9-(2-hydroxyethyl)adenine and about 1.2-2-2 molar equivalents of sodium alkoxide.

In an embodiment, synthesis of 9-[2-(diethylphosphonomethoxy)-ethyl]adenine, shown in Diagram A, Step 3, is described as follows. In a reactor having an inert atmosphere, e.g., nitrogen, a slurry of 9-(2-hydroxyethyl)adenine (1.0 kg) and DMF (4.79 kg) is warmed to about 130° (125-135°) for 30-60 minutes. The reactor contents are rapidly cooled with vigorous agitation to about 25° (20-30°) and sodium *tert*-butoxide (0.939 kg) is added in portions over about 1-3 hours while maintaining vigorous agitation and the contents temperature at about 25° (20-30°). The agitation and temperature is maintained for about 15-45 minutes after all sodium tert-butoxide has been added. Then the reactor contents are cooled to about -10° (-13 to 0°) and a solution of diethyl *p*-toluenesulfonyloxymethyl-phosphonate (2.25 kg on a pure basis) in DMF (1.22 kg) is added over about 5-10 hours. The mixture is kept at about -5° (-10 to 0°) until the reaction is complete, which is typically about 0.5-2 hours after the final portion of diethyl p-toluenesulfonyloxymethylphosphonate has been added. Reaction completion is monitored by HPLC (not more than 3% 9-(2-hydroxyethyl)adenine remaining). Glacial acetic acid (0.67 kg) is added, with the pot temperature controlled to no more than 20°. The mixture at about 22° (15-25°) is agitated for about 15-45 minutes. The quenched mixture is concentrated *in vacuo* until distillation stops and the contents are then cooled to below 40°. Dichloromethane (16.0 kg) is added and the contents at 20° (15-25°) are agitated for at least 1 hour. If the DMF content versus total solids (NaOTs (sodium tosylate), NaOAc, Et₂PMEA) is greater than 20% (by ¹H NMR) the mixture is concentrated *in vacuo* until distillation stops, the contents are cooled to below 40°C, dichloromethane (16 kg) is added and the reactor contents at about 20° (15-25°) are agitated for at least 1 hour. Diatomaceous earth (0.5 kg) is added and the contents, which are at about 20° (15-25°), are agitated for at least 1 hour. The solids are removed by filtration and rinsed 3 times with CH₂Cl₂ (about 1 kg each). The filtrate and rinses at no more than 80° are concentrated *in vacuo* until distillation stops, the reactor contents are cooled to below 40°, dichloromethane (5.0 kg) is added to the residue and the contents at about 25° (20-40°) are agitated to dissolve the solids. The resulting solution at no more than 80° is concentrated in vacuo until distillation stops. Dichloromethane (7.0 kg) is added and the contents at about 25° (20-40°) are agitated to dissolve the solids. If the DMF content compared to diethyl PMEA is greater than 12%, the mixture at no more than 80° is concentrated *in vacuo*, the contents are cooled to below 40°, dichloromethane (7.0 kg) is added and the contents at about 25° (20-40°) are agitated to dissolve the solids. The mixture is washed with water (0.8 kg) at about 25° (22-30°) by agitating for about 15-45 minutes. The phases are allowed to separate for 4 hours and the phases are then separated. The aqueous phase is back-extracted twice with dichloromethane (1.5 kg per wash) by agitation for about 15-45 minutes with the solution maintained at about 25° (22-30°), followed by allowing the phases to separate for at least 2 hours. The combined organics at no more than 80° are then concentrated *in vacuo* until distillation stops. Toluene (3.0 kg) is added, agitated at about 25° (22-30°) for about 15-45 minutes and the resulting mixture at no more than 80° is azeotroped *in vacuo.* Toluene (3.0 kg) is added and the mixture is heated to about 80° (75-85°), agitated for about 15-45 minutes, cooled to below 30° over about 60-90 minutes and then cooled to about 0° (-3 to 6°). After at least 12 hours at about 0° with slow agitation, the resulting slurry is filtered and the filter cake is rinsed three times with cold (about 0-6°) toluene (about 0.2 kg per rinse). The wet cake is dried *in vacuo* at about 50° (35 to 65°) and the dried product is milled. Product drying is monitored for water removal (no more than 0.3% water detected by KF titration). The inert atmosphere is maintained throughout step 3.

### Methods for PMEA Synthesis

In an embodiment, synthesis of PMEA, shown in Diagram A, Step 4, is described as follows. In a reactor having an inert atmosphere, e.g., nitrogen, a mixture of diethyl PMEA (1.00 kg), acetonitrile (2.00 kg), and bromotrimethylsilane (1.63 kg) is heated to and maintained at reflux for about 1-3 hours with agitation, until the reaction is complete. Reaction completion is monitored by ³¹P NMR or HPLC (no diethyl PMEA and no more than 2% monoethyl PMEA detected). The solution at ≤ 80°C is distilled *in vacuo* to a semi-solid, which is taken up in water (2.00 kg) and warmed to about 55°C (52-58°C) for about 30-60 minutes with agitation to dissolve all solids. The resulting mixture is cooled to about 22°C (19-25°C), adjusted to pH 3.2 with aqueous sodium hydroxide, the contents are heated to about 75°C (72-78°C) until the consistency thins (about 15-120 minutes), cooled to about 3°C (0-6 °C), and stirred for at least 3 hours (3-6 hours). The slurry is filtered and the filter cake is rinsed with water (1.00 kg). The wet cake is suspended in water (3.75 kg) and the suspension is heated to about 75°C (72-78°C) with vigorous stirring. After stirring for about 2 hours, the slurry is cooled to about 3°C (0-6 °C) and stirred for at least another 2 hours. The slurry is filtered and the filter cake is rinsed sequentially with two portions of water (0.50 kg per rinse) and two portions of acetone (1.00 kg per rinse). The isolated solid is dried *in vacuo* at no more than about 90°C to a low water content (no more than 0.5% water detected by KF titration), to provide PMEA as white crystals. The, product is milled to a fine particle size.

### Methods for AD Synthesis

An exemplary method to prepare AD comprises suspending 1 molar equivalent of PMEA in a volume of about 5.68-56.8 equivalents of NMP/equivalent PMEA and, after one suspends the PMEA, adding about 2-5 molar equivalents, often about 2.5-3.5, usually about 3 molar equivalents, of triethylamine ("TEA") to the solution using mild to moderate agitation. One then adds about 3-6 molar equivalents, often about 4.5-5.5 molar equivalents, usually about 5 equivalents, of chloromethyl pivalate to obtain a reaction mixture. We usually prepare the reaction mixture at room temperature. One heats the reaction mixture to maintain a temperature of less than 66°, typically about 28-65°, usually between about 55-65° for about 2-4 hours to conduct the reaction. The time needed to heat the reaction mixture to about 28-65° is not critical and can vary depending on the reaction mixture volume and the capacity of the apparatus used to heat the mixture. Mild or moderate agitation maintains solids in suspension during the reaction and this minimizes extensive splashing of the reactants in the reaction vessel. This method results in a product comprising AD produced by the process of reacting the listed reactants, typically under the given conditions.

In an embodiment, conversion of PMEA to AD, shown in Diagram A, Step 5, is described as follows- In a reactor having an inert atmosphere, e.g., nitrogen, a mixture of 1-methyl-2-pyrrolidinone (3.15 kg), PMEA (1.00 kg), triethylamine (1.11 kg), and chloromethyl pivalate (2.76 kg) is heated to about 60 ± 3°C (no more than 66°C) and stirred using moderate agitation for ≤ 4 hours (1-4 hours) until the reaction is complete, as indicated by ³¹P NMR or HPLC (no more than 15% mono(POM)PMEA). The mixture is diluted with isopropyl acetate (12.00 kg), cooled to 25 ± 3°C, and agitated for about 30 minutes. The solids are removed by filtration and washed with isopropyl acetate (5.0 kg). The combined organics are washed twice with water (3.70 kg per wash) by moderately agitating the mixture at a mixture temperature of 25 ± 3°C for about 15-45 minutes. The combined aqueous washes are back-extracted twice with isopropyl acetate (4.00 kg per extraction) at a mixture temperature of 25 ± 3°C by agitation for 15-45 minutes. The combined organics at 25 ± 3°C are washed with water (1.80 kg) by agitation for 15-45 minutes and then the organics at about 35 ± 5°C (no more than 40°C) are concentrated *in vacuo* to approximately 40% of the original volume. After a polishing filtration (1 µm filter), and a rinse forward with 1.5 kg of isopropyl acetate, the concentration of the organics *in vacuo* is resumed until a pale oil remains the organics at about 35 ± 5°C (no more than 50°C). The oil typically comprises about 6-45% AD, usually about 30-42%.

## Claims

1. A method for preparing 9-[2-(diethylphosphonomethoxy)ethyl]-adenine comprising the step of contacting sodium alkoxide and 9-(2-hydroxyethyl)-adenine.

2. The method of claim 1, wherein 1.2-2.2 molar equivalents of sodium alkoxide per molar equivalent of 9-(2-hydroxyethyl)-adenine are used.

3. The method of claim 1 or 2, wherein the sodium alkoxide and 9-(2-hydroxyethyl)-adenine are contacted in dimethylformamide as solvent.

4. The method of any one of the preceding claims wherein the sodium alkoxide and 9-(2-hydroxyethyl)-adenine are contacted at a temperature of 20 to 30 °C.

5. The method of any one of the preceding claims wherein the sodium alkoxide is selected from sodium t-butoxide and sodium i-propoxide.

6. The method of any one of the preceding claims further comprising the step of reacting the obtained product with diethyl-toluenesulfonyloxymethylphosphonate.

## Patentansprüche

1. Verfahren zur Herstellung von 9-[2-(Diethylphosphonomethoxy)ethyl]-adenin, bei dem man Natriumalkoxid und 9-(2-Hydroxyethyl)-adenin in Kontakt bringt.

2. Verfahren nach Anspruch 1, bei dem man 1,2 - 2,2 Moläquivalente Natriumalkoxid pro Moläquivalent 9-(2-Hydroxyethyl)-adenin verwendet.

3. Verfahren nach Anspruch 1 oder 2, bei dem man das Natriumalkoxid und 9-(2-hydroxyethyl)-adenin in Dimethylformamid als Lösungsmittel in Kontakt bringt.

4. Verfahren nach einem der vorhergehenden Ansprüche, bei dem man das Natriumalkoxid und 9-(2-Hydroxyethyl)-adenin bei einer Temperatur von 20 - 30 °C in Kontakt bringt.

5. Verfahren nach einem der vorhergehenden Ansprüche, bei dem das Natriumalkoxid unter Natrium-t-butoxid und Natrium-i-propoxid ausgewählt ist.

6. Verfahren nach einem der vorhergehenden Ansprüche, bei dem man außerdem das erhaltene Produkt mit Diethyltoluolsulfonyloxymethylphosphonat umsetzt.

## Revendications

1. Méthode de préparation de 9-[2-(diéthylphosphonométhoxy)éthyl]-adénine comprenant l'étape de mettre en contact de l'alcoolate de sodium et de la 9-(2-hydroxyéthyl)-adénine.

2. Méthode de la revendication 1, où on utilise 1,2-2,2 équivalents molaires d'alcoolate de sodium par équivalent molaire de 9-(2-hydroxyéthyl)-adénine.

3. Méthode de la revendication 1 ou 2, où l'alcoolate de sodium et la 9-(2-hydroxyéthyl)-adénine sont mis en contact dans le diméthylformamide comme solvant.

4. Méthode de l'une quelconque des revendications précédentes où l'alcoolate de sodium et la 9-(2-hydroxyéthyl)-adénine sont mis en contact à une température de 20 à 30°C.

5. Méthode de l'une quelconque des revendications précédentes où l'alcoolate de sodium est sélectionné parmi t-butoxyde de sodium et i-propoxyde de sodium.

6. Méthode de l'une quelconque des revendications précédentes comprenant de plus l'étape de faire réagir le produit obtenu avec du diéthyl-toluènesulfonyloxyméthylphosphonate.
